# FASCICULE DE BREVET EUROPEEN

(11) **EP 1 681 973 B1**
(45) Date de publication et mention de la délivrance du brevet: **19.08.2009**
(21) Numéro de dépôt: 04805413.4
(22) Date de dépôt: 09.11.2004
(51) Int. Cl.: A47K 17/00

(54) **ECRAN PROTECTEUR D' HYGIENE FEMININE**
HYGIENESCHUTZ FÜR FRAUEN
FEMININE HYGIENE PROTECTIVE SHIELD

(30) Priorité: 12.11.2003 FR 0313241; 13.04.2004 FR 0403832
(43) Date de publication de la demande: 26.07.2006
(73) Titulaire: Bazelaire, Marylise, 83119 Brue Auriac (FR)
(72) Inventeur: Bazelaire, Marylise, 83119 Brue Auriac (FR)
(86) Numéro de dépôt international: PCT/FR2004/002870
(87) Numéro de publication internationale: WO 2005/048803

(56) Documents cités:
- DE-U- 20 016 954
- US-A- 4 756 029

## Description

La présente invention concerne un écran protecteur pour l'hygiène intime féminine. Un écran protecteur est connu du document DE 20016954 U.

Cet écran protège la partie externe de l'appareil génital féminin, en particulier la vulve, des éclaboussures sur les parois du récipient, lors des écoulements urinaires.

En effet, ces éclaboussures peuvent être le véhicule de champignons, virus, bactéries responsables d'infections bactériennes ou virales ou de mycoses, en particulier d'infections urinaires ou vaginales.

Le récipient en question est, le plus souvent, la cuvette des toilettes et ledit écran protecteur protège également des éclaboussures d'eau retenue dans le siphon.

Cette protection s'interpose entre l'appareil génital féminin externe et le récipient, quel qu'il soit, où la femme urine, en l'occurrence la cuvette des toilettes.

Cet écran est indispensable pour la personne soucieuse d'une bonne hygiène.

De plus, les femmes ayant des problèmes de vessie et ne pouvant retenir leur jet y trouveront aisance et soulagement.

Les personnes étant plus vulnérables aux attaques microbiennes dans certaines situations (ayant subi une opération des voies génitales ou à la suite d'un accouchement, d'une épisiotomie, etc...) y trouveront un certain confort dans sa sécurité.

De par sa praticité, les petites filles peuvent également l'utiliser.

L'invention concerne ainsi un écran protecteur suivant la revendication 1.

L'utilisatrice peut ainsi facilement, et de façon hygiénique, appliquer le passage voilé en regard de son appareil génital féminin et le maintenir dans la position relative voulue. Le voile constitue ainsi un filtre anti-retour d'éclaboussures.

L'écran est constitué de matériaux biodégradables.

On peut ainsi, après usage, le jeter directement pour l'évacuer par le conduit des toilettes.

Le voile peut comporter une entaille pour faciliter l'écoulement de l'urine.

Dans la mesure où les lèvres de l'entaille restent sensiblement jointives, le voile remplit sa fonction d'écran.

Le support présente un contour externe sensiblement elliptique.

Il présente ainsi une forme ergonomique.

Dans une forme de réalisation, le passage présente une embouchure munie d'un anneau de renfort agencé pour maintenir le voile.

Le voile peut être enduit d'un désinfectant.

Dans une forme de réalisation, l'écran comporte un agglomérat de fibres constituant, d'un seul tenant, le voile et l'appendice de saisie.

La fabrication de l'écran peut ainsi s'effectuer directement par moulage.

L'écran présente de préférence un contour externe de dimension maximale inférieure à 15 centimètres dans une direction longitudinale d'extension et inférieure à 10 centimètres dans une direction transversale.

Le support peut en particulier être sensiblement plat, c'est-à-dire se présenter sous la forme d'une languette ou plaquette à contour adapté, obtenue par moulage ou par découpe d'une plaque, par exemple en carton ou en matériau plastique.

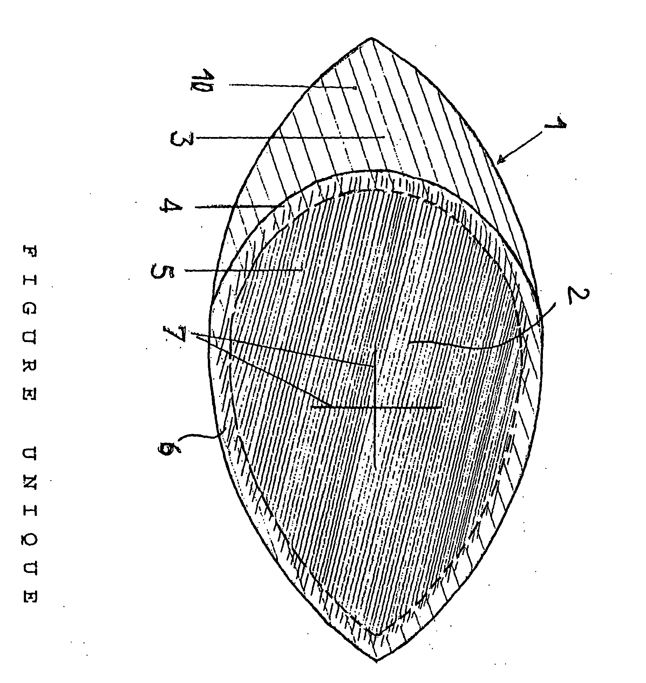

L'invention sera mieux comprise à l'aide de la description suivante d'une forme de réalisation d'un écran protecteur selon l'invention, en référence à la figure unique annexée.

La figure représente un écran protecteur 1 pour l'hygiène intime féminine, destiné à protéger l'appareil génital féminin contre des éclaboussures provenant des parois d'un récipient dans lequel urine une femme, l'écran 1 comprenant un voile écran de protection 5 monté transversalement à un passage 2 pour l'urine ménagé dans un support 10 portant le voile 5 et comportant un appendice de saisie 3.

Le support 10 est ici plat, c'est-à-dire sous forme de plaquette ou languette en carton présentant un contour externe sensiblement elliptique. Le passage 2 est ménagé dans une zone centrale ainsi qu'une zone d'extrémité du support 10, et l'appendice de saisie 3 forme une zone d'extrémité du support 10, constituant une excroissance d'une piste d'embouchure 6 limitant le passage 2.

Le contour externe du support 10 est ici de dimension maximale inférieure à 15 centimètres dans une direction longitudinale d'extension et inférieure à 10 centimètres dans une direction transversale.

Le voile 5 est porté par le support 10, et précisément ici par l'embouchure 6, qui est munie, dans cet exemple, d'un anneau de renfort 4 agencé pour maintenir le voile 5. L'anneau de renfort 4 peut être un simple anneau plat ou bague plaqué et collé sur l'embouchure 6, avec interposition du bord du voile 5 qui est ainsi fixé. En variante, il peut s'agir d'une bague comportant une rainure externe agencée pour venir emprisonner les deux surfaces opposées d'embouchure 6 respectivement situées sur la grande face ici visible et sur la grande face opposée, le bord du voile 5 étant glissé dans cette rainure pour y être ainsi fixé.

Le voile 5 comporte ici une entaille 7, présentant dans cet exemple une forme de croix, pour faciliter l'écoulement de l'urine, et il est enduit d'un désinfectant.

Les matériaux ici utilisés sont biodégradables.

En variante, l'appendice de saisie 3, voire même tout le support 10, et le voile 5 sont formés, d'un seul tenant, par un agglomérat de fibres ou particules, la densité d'implantation des fibres ou particules étant réduite localement pour former la zone du passage 2 à voile 5, par rapport à la densité du support 10, c'est-à-dire la zone d'embouchure 6 et la zone de saisie 3.

## Revendications

1. Ecran protecteur en matière biodégradable pour l'hygiène intime féminine de forme ergonomique à maintenir en regard de l'appareil génital, destiné à protéger cet appareil génital externe des éclaboussures provenant des parois d'un récipient dans lequel urine une femme, l'écran comprenant un voile écran de protection (5) constituant un filtre anti-retour d'éclaboussures monté transversalement à un passage (2) pour l'urine ménagé dans un support (10) présentant un contour externe sensiblement elliptique auquel ledit voile (5) est fixé et comprenant par excroissance un appendice de saisie (3) permettant de maintenir cet écran sous la vulve dans la position relative voulue.

2. Ecran selon la revendication 1 dans lequel le voile (5) comporte une entaille (7) pour faciliter l'écoulement de l'urine, les lèvres de l'entaille restant sensiblement jointives.

3. Ecran selon l'une des revendications 1 à 2 dans lequel le passage (2) présente une embouchure munie d'un anneau de renfort ou bague (4) agencé pour maintenir le voile (5) par interposition, emprisonnant celui-ci par deux faces d'embouchures (6) opposées pouvant former rainure.

4. Ecran selon l'une des revendications 1 à 3 dans lequel le voile (5) est enduit d'un désinfectant.

5. Ecran selon l'une des revendications 1 à 4 comportant un agglomérat de fibres ou particules constituant, d'un seul tenant, par moulage le support (10), le voile (5) et l'appendice de saisie (3).

6. Ecran selon l'une des revendications 1 à 5 présentant un contour externe de dimension maximale inférieure à 15 centimètres dans une direction longitudinale d'extension et inférieure à 10 centimètres dans une direction transversale.

7. Ecran selon l'une des revendications 1 à 6 dans lequel le support (10) en forme de languette ergonomique est sensiblement plat.

## Claims

1. An ergonomically shaped protective screen for intimate female hygiene, made of biodegradable material to protect the external genital organ from splashes from the sides of a receptacle into which a woman urinates, the screen comprising a protection voile (5) constituting an anti-return splash filter fitted transversely to a passage (2) for urine, arranged in a support (10) possessing an essentially elliptical external contour, to which the voile (5) is fastened and also comprising a gripping appendix (3) in the form of an excrescence making it possible to hold the said screen in the desired position under the vulva.

2. A protective screen according to Claim 1, in which the voile (5) comprises an indentation (7) to facilitate the flow or urine, the lips of the indentation substantially abutting.

3. A protective screen according to one of the Claims 1 to 2 where the passage (2) has an opening fitted with a reinforcing circle or ring (4) so arranged as to hold the voile (5) by means of interposing itself and seizing the latter by two opening faces (6) opposite one another and able to form a groove.

4. A protective screen according to one of the Claims 1 to 3 in which the voile (5) is coated with a disinfectant.

5. A protective screen according to one of the Claims 1 to 4 comprising an agglomerate of fibres or of particles, constituting the support (10) moulded in one piece, the voile (5) and the gripping appendix (3).

6. A protective screen according to one of the Claims 1 to 5 with an external contour of maximum size of less than 15 centimetres in longitudinal direction of extension and of less than 10 centimetres in transverse direction.

7. A protective screen according to one of the Claims 1 to 6, in which the support (10) in the form of an ergonomic tongue is substantially flat.

## Patentansprüche

1. Ergonomisches Schutzschild aus biologisch abbaubarem Material für die Intimhygiene der Frau, der vor die Genitalien zu halten ist und dazu dient diese vor Spritzern aus den Wänden eines Behälters zu schützen in den die Frau uriniert. Das Schild enthält einen Schutzschleier, (5) der einen Rückspritzschirm bildet, der quer zum Urinablass (2) montiert ist, welcher in einem Rahmen (10) ausgespart ist und deutlich elliptische äußere Konturen aufweist und an dem obengenannter Schutzschleier (5) befestigt wird und an dem ein Greifansatz (3) ausgestülpt ist, der das Festhalten dieses Schildes in der gewünschten betreffenden Position unter der Scham ermöglicht.

2. Schild gemäß Vorgabe 1, in der der Schleier (5) einen Einschnitt (7) aufweist um den Urinausfluss zu erleichtern, die Lippen des Einschnittes bleiben dabei deutlich aneinandergesetzt.

3. Schild gemäß einem der Ansprüche 1 bis 2 in dem der Urinablass (2) eine durch einen Ring oder Reif (4) verstärkte Mündung aufweist, die so angebracht ist, dass sie den Schleier (5) durch Einfügung festhält, wobei die beiden gegenüberliegenden Mündungsseiten eine Nut bilden können.

4. Schild gemäß einem der Ansprüche 1 bis 3, in dem der Schleier (5) mit einem Desinfektionsmittel behandelt wird.

5. Schild gemäß einem der Ansprüche 1 bis 4 mit einem Faser- oder Teilchengemisch, der in einem Stück durch Formarbeit den Rahmen (10), den Schleier (5) und den Greifansatz (3) bildet.

6. Schild gemäß einem der Ansprüche 1 bis 5, mit einer Außenlinie von weniger als 15 Zentimeter in Richtung einer Längsvergrößerung und weniger als 10 Zentimeter in Querrichtung.

7. Schild gemäß einem der Ansprüche 1 bis 6, bei dem der Rahmen (10) in Form eines ergonomischen Anhängsels deutlich flach ist.
